# EUROPEAN PATENT SPECIFICATION

(11) **EP 4 420 655 B1**
(45) Date of publication and mention of the grant of the patent: **12.11.2025**
(21) Application number: 23158045.7
(22) Date of filing: 22.02.2023
(51) Int. Cl.: A61K 8/73, D21H 11/12, D21H 21/18, D21C 5/00, D21H 17/24, D21H 27/00

(54) **BACTERIAL CELLULOSE BIO-COMPOSITE AND AN ARTICLE COMPRISING SAID BIO-COMPOSITE**
BAKTERIELLER CELLULOSEBIOVERBUNDSTOFF UND ARTIKEL MIT DIESEM BIOVERBUNDSTOFF
BIOCOMPOSITE DE CELLULOSE BACTÉRIENNE ET ARTICLE COMPRENANT LEDIT BIOCOMPOSITE

(43) Date of publication of application: 28.08.2024
(73) Proprietor: Malai Biomaterials Design s.r.o., 07231 Vinne (SK)
(72) Inventor: Gombosova, Zuzana, 07231 Vinne (SK); Chempodil, Susmith, 686003 Veloor - Kottayam (IN)
(74) Representative: Majlingová, Zuzana

(56) References cited:
- WO-A1-89/01074
- US-A- 5 207 826
- US-A1- 2021 114 334

## Description

### TECHNICAL FIELD

The present invention relates to biodegradable biocomposites based on Bacterial cellulose and the method of manufacturing of such biocomposite materials.

More particularly the present invention relates to natural, compostable material that, inter alia, can have the appearance of leather.

It should be understood that the use of the novel material should not be limited to leather substitute as herein described.

### BACKGROUND ART

Demand for sustainable and biodegradable leather alternatives in the current market is high. Materials most commonly offered as a replacement for leather contain polyvinylchloride (PVC) and polyurethane (PU) - both non-biodegradable, difficult to recycle and releasing high levels of volatile compounds (VOC) to the environment during manufacturing as well as usage stage. Industries like fashion, furniture, footwear and automotive are all actively looking for better and more sustainable alternatives.

Cultivation of Bacterial cellulose on water from mature coconuts is widespread practice in many countries of South - East Asia including Indonesia, Vietnam, Thailand and Philippines where the pellicle is commonly called Nata de Coco.

Banana plant is cultivated all around tropics mainly for fruit. The stem of the plant, however, is mainly left to rot once the fruit has been harvested. Strong, lustre fibre can be extracted from the outer layers of banana plant stem with similar appearance to silk. The banana stem fibres used to make this novel natural biocomposite material described are a by-product of banana fruit harvest. Income of banana farmers would be enhanced by fibre extraction process needed for the production of this material.

The document WO8901074A1 discloses a method of bonding a fibrous wet laid nonwoven fabric-like product using bacterial cellulose as a binder. From 1-30 % of the bacterial cellulose may be added to a water slurry of other sheet forming fibres as a binder. Preferably 20 % or less bacterial cellulose is used since higher amounts retard drainage rates significantly. However, due to the different method of production, final products lack appearance, texture and properties similar to animal leather and describes a very general product that can be achieved using this method and crucial details that define the variability of the outcomes are missing in the description.

The document US5207826A discloses a binding agent comprising fibre strands of microbially produced cellulose. The cellulose is synthesized by certain strains of microorganisms of the genus Acetobacter grown under agitated culture conditions to produce fine, interconnected fibre stands forming a three-dimensional reticulated structure. The binding agent is effective for binding fibres and fragments of material, such as natural and treated wood fragments and particles as well as synthetic materials, and it imparts wet and dry tensile strength to the bound material. However, no specifications are given on character of the product outcome from the process. It is suggested as a manufacturing method for hydraulic mulches where bacterial cellulose substitutes other available tackifiers as a binder. This invention does not attempt to create a leather like material. We get no information about stitchability, etc.

The aim of this invention is to achieve a sustainable and biodegradable biocomposite material based on bacterial cellulose and natural fibres that is strong, durable and flexible and possesses an appearance, texture and tensile strength (TS) values similar to animal leather.

### SUMMARY OF THE INVENTION

Bacterial cellulose is a natural compound produced / fermented by bacterium such as *Acetobacter* strains that can be grown on several different types of media - with a possibility of using agricultural waste streams. Natural fibres from banana stem, sisal and hemp are added to strengthen the pellicle and add body to the material along with natural gums and starches.

Biocomposite materials based on these ingredients and processed in manner described in the invention are strong, flexible, remain biodegradable and can replace leather in applications like accessories, wallets, light footwear and stationery - non load bearing applications. They can be processed into products using same or similar techniques as work with animal leather requires (stitching, gluing, skiving, edge burnishing, etc.)

Bacterial cellulose biocomposite according to the present invention is obtained by the following manufacturing process:
I.
   a) cutting the banana fibre, the hemp fibre and the sisal fibre to a length of 2 to 10 cm, preferably to length of 6 to 8 cm;
   b) delignification of the banana fibre, the hemp fibre and the sisal fibre (each separately), either enzymatically or in the alkaline solution;
   c) removing impurities from the delignified banana fibre, the hemp fibre and the sisal fibre, preferably washed with water;
   d) pulping the banana fibre, the hemp fibre and the sisal fibre (each separately) mechanically or enzymatically to a length 5 - 50 mm;
II. pulping a bacterial cellulose to a length of 10-100 µm; and if necessary, adjusting pH of the pulp to 5 to 7, preferably to 5.5;
III. mixing the components obtained in the steps I. and II. in amounts:
   - 35 to 60 wt. % of banana pulp,
   - 20 to 45 wt.% of hemp pulp,
   - 5 to 20 wt. % of sisal pulp, and
   - 10 to 20 wt. % of bacterial cellulose pulp;
IV. adding 0,2 to 20 wt. % of polysaccharide cationic gum into the mixture, preferably 3 to 10 wt. %, the most preferably 5 wt. %;
V. stirring the mixture until homogenous dispersion of the fibres in the matrix (matrix being the binding part of the composite) is achieved;
VI. diluting the mixture with water to achieve 0.3 wt. % to 0.03 % of composite solution and pouring the solution of fibres and matrix in a sheet forming mould having a water-permeable bottom, and left there until the water is filtered out through the bottom (ideally using a vacuum suction) until 25 to 15 % of the initial volume of the composite solution is achieved, preferably 15 %;
VII. further dewatering the sheet until the content of water is less than 80% of the sheet weight,
VIII. drying the sheet at the temperature up to 60 °C, preferably 20 to 50 °C, until the overall moisture content in the sheet is lower than 10 wt.%, preferably 8-9 wt.%;
IX. treating the sheet with 5 to 30 wt. % plasticizer and humectant with respect to dry weight of sheet from both sides and letting the sheet absorb this solution, until the moisture content of the sheet increases to 20-30 wt. %;
X. mechanical manipulation - multiple stretching and folding of the sheet in order for material to be pliable and without warps (wraps can be created during uneven drying process);
XI. treating both sides of the sheet with 10 - 30 wt.%, preferably 12 wt.%, water solution of sizing improving the tactile properties of surface, preferably the sizing is 90-99% pure glucomannan, and drying the sheet;
XII. sheet is further mechanically softened and texturized, preferably using a graining machine,
XIII. performing step XI, at least once;
XIV. the dry sheet is optionally treated with hydrophobic agent.

I.a)
Banana, hemp and sisal fibres in their freshly extracted or dry extracted form can be used. Fibres of pre-scutching form or scutched fibres can be used, not finer than that. The respective fibres should be cut to the length of 2 to 10 cm, preferably to length of 6 to 8 cm.

The fibre length is important in order to achieve material of significant tensile and tear strength and for the ease of processing. If fibres are longer than 10 cm they will very likely get entangled in the fibre beating process and therefore won't form a uniform mat during the sheet forming process. If fibres are shorter than 2 cm the material's tear strength will be compromised.

I.b)
Delignification, the process of extracting lignin from plant sources, can be done using a variety of methods.

The fibre is brought to a boil in 4 to 15% alkali water solution and left to cool to at least 28°C or less, preferably to a room temperature (20-28 °C).

Alternatively, the fibre can be delignified by enzymatic process (using laccase enzyme for example).

I.c)
Fibres are separated from the alkali solution - drained, and subsequently washed with water until the water runs clear and impurities are completely removed (until the pH of the water is 6 to 7). Removing impurities to the large extent improves the aesthetic qualities of the finished material, its homogenous dyeability and even bonding of fibres. This can be done manually or using a washing machine (to save up water consumption and effort).

I.d)
The fibre can be pulped either mechanically or enzymatically. Either using any of the conventional mechanical pulping equipment or using enzymes, in either way maintaining the length of the fibre between 5 - 50 mm (to ensure high strength of the material and easy processability).

Fibres can be pulped using Hollander beater, kollergang beater or screw extrusion pulper. Each fibre type should be processed separately. The degree of fibrillation should be less than kraft paper or paper. Degree of fibrillation can be checked and standardised using Schopper riegler tester.

II.
Bacterial cellulose can be obtained by fermentation process of Acetobacter strain of bacteria grown preferably on water from mature coconut / secondary coconut milk media. Fermentation methods can be either static fermentation methods or agitated fermentation methods.

Bacterial cellulose is pulped into a pulp having particle size 10-100 µm.

Bacterial cellulose can be pulped using high speed mixers and/or ultrasonic homogenisers.

The pH is checked and adjusted to range from 5 to 7, preferably 5.5. This is important for the subsequent dyeing processes where pH value plays a role and can directly influence the colour shade achieved from using a particular dye.

III.
Banana, hemp and sisal fibres are taken in a ratio of 35-60%, 20-50%, 5-20% from their respective surface density (ratio can be altered within range no greater than ±10% depending on desired strength, softness and tensile strength properties). Depending on the final colour option of the sheet the fibres can be dyed at this stage. Natural plant-based dyes can be used to dye the pulp, extracts from indigo, marigold, brazilwood, etc.

Prepared pulp of bacterial cellulose forms 10-20% of the formulation. If higher than that, the drainage rate is very slow without having any direct impact on increasing the tear or tensile strength.

IV.
Polysaccharide cationic gum (such as e.g. guar gum) is added to the formulation at max 8% of the weight of the sheet. Adding polysaccharide cationic gum improved tensile strength and elastic elongation of the final product. If the ratio of the gum is above 8% the drainage time is too long however no significant improvement in tensile strength property occurs in comparison with 5%.

V.
The mixture is agitated for 15 to 60 min., usually 20-30 min. is enough to ensure homogenous dispersion of the fibres in the matrix.

Optionally, pH is adjusted to 5-6, depending on the desired colour outcome of the sheet. Indigo coloured sheets need pH above 10.

VI.
Solution is diluted to 0.3 wt. % to 0.03 wt. % with addition of water, preferably to 0.3 wt. %. Optionally, pH can be adjusted to 5-6, depending on the desired colour outcome of the sheet. Indigo coloured sheets need pH above 10.

The solution of fibres and matrix is poured in a sheet forming mould with a water permeable bottom part. The water is filtered out through the bottom mesh and water recycled in the next sheet making process, while fibres arrange themselves freely into an organic web structure.

A sheet is considered ready to remove from the mould when most of the water is removed to 1.5% content of the initial volume. The weight of the sheet at that point would be around 2-3 kg and its thickness 1.5 to 2 cm. The solid content of the sheet in this stage is 5-10% - rest is water.

VII.
Sheet is further dewatered until the content of water is less than 80% of the sheet weight. Preferably in 80T hydraulic press (pressure 0.3-1 MPa) to thickness of approximately 5 mm. It is placed on a mesh frame and transported to a drying facility.

VIII.
The sheets require drying temperatures closest to the conditions of drying under the sun - 25-50°C. At these temperatures fibres and matrix interact with each other, crosslink and form stronger bonds which is essential for achieving higher tensile and tear strength of the material. The temperature should not be higher than 80 °C. It takes approximately 6-8 hours to lower the overall moisture content in the sheet under 10 wt.%. The overall moisture content in the sheet should be preferably 8-9 wt.%. Drying at low temperatures (25 to 40°C) decreases brittleness and improves strength of the final material.

VIII a)
Sheet can be treated with 2 wt.% to 20 wt.% of carboxylic acid with a long chain, or its derivatives, preferably stearic acid. Preferably the sheet is immersed in a stearic acid hot bath (60°C to 100°C) for 1 to 4 hours. Or the dissolved stearic acid is blended into a biocomposite slurry and heat treated post drying process.

This step is optional. It can be used in case it is desired to improve water resistance and tensile strength of the final sheet. stearic acid also increases abrasion resistance and softness of the final product.

IX.
Once sheets are dried, they are hard, rigid and often warped. The area of the sheet would have shrunk by 15-30%, depending on the ratio of the fibres and bacterial cellulose. Therefore, the sheet is treated with 5 to 30 wt. % humectant / plasticizer with respect to dry weight of sheet and letting the sheet absorb the solution, until the moisture content of the sheet increases to 20-30 wt. %.

A humectant is a substance with affinity to form hydrogen bonds with molecules of water, it attracts and retains the moisture in the air nearby via absorption, drawing the water vapor into or beneath the object's surface. The suitable humectants can be for example: Propylene glycol, hexylene glycol, and butylene glycol, aloe vera gel, alpha hydroxy acids such as lactic acid, glyceryl triacetate, polymeric polyols such as polydextrose, quillaia, sodium hexametaphosphate E452i, sugar alcohols (sugar polyols) such as glycerol, sorbitol, xylitol, maltitol, urea. Preferably, a humectant can be diluted with water, isopropylalkohol or ethanol to 10 to 30 wt.%. solution of humectant, to improve absorbency into the material. The humectant solution can be applied on the sheets surface by method of spraying or blade coating or fogging (ULV sprays) or by dipping the material. The humectant solution is applied to both sides of the sheet. The Sheet is left to dry.

X.
The sheet is then repeatedly folded and stretched to remove the warps. Folding can be performed by hand or by a folding machine. The sheet is preferably folded and stretched at least two times in 4 directions - horizontally, vertically, and diagonally. Preferably, the respective folds are spaced 3 - 30 mm apart, preferably 5-20 mm, most preferably 5-10 mm. This process was specifically developed for manufacturing of this material.

X a)
The sheet can be then exposed to a pressure of 1- 10 MPa, preferably 1 - 5 MPa, most preferably 3 MPa at the temperature 60 to 120°C, preferably at the temperature 80°C, preferably for 30-180 s. A horizontal press or a calender machine can be preferably used. This step is optional and improves the surface properties of the sheet (smoothens of the surface) and compresses the material into higher density that benefits moisture resistance, durability and abrasion resistance properties.

XI.
The suitable sizing composition for tactile properties improvement that can be used in the step XI are: glucomannan extract, *Pseuderanthemum carruthersii* (Seem.), guillaumin extract, pectins, beta-glucans, chitosan, and the group of galactomannans. Preferably a coating is prepared using glucomannan (99% purity starch extract from konjac root) powder and water by dispersing the powder in cold water and slowly bringing it to boil. The coating is applied to the surface of the sheet from both sides preferably using a blade coating method. Glucomannan positively influences material's textural memory, strength and water-resistance.

Glucomannan is a water-soluble polysaccharide. It is a hemicellulose component in the cell walls of some plant species.

XII.
Once dry, the material is softened and texturized preferably using a boarding / graining type of machine. This process makes the material more supple, flexible and creates its signature grain texture. The machine comprises a series of rollers and a dull blade in between, the sheet is passed through the machine in at least two directions, preferably in all 4 directions, preferably 50 to 90 times in each direction or until desired level of flexibility and texture is achieved. Material can be also tumbled or embossed using roller or plate pressing or calendered.

XIII.
The sheet surface is coated with the glucomannan coating. It can be calendared again at this point using pressure of 1-5 MPa and temperature 50 - 80 °C.

Additional coating comprising of chitosan and carboxylic acid can be applied to the material to improve moisture retention and as antifungal treatment.

XIV.
Afterwards, the sheet can be optionally treated with a hydrophobic agent to improve its hydrophobic properties.

The dry sheet is preferably coated with natural wax emulsion or saturated fatty acid-based oil or combination thereof. Preferably, the coating is applied at least twice from each side and left to dry completely in between each coat. Biopolymers can be considered for this step too, especially PLA, PHB , agar based and combination of both although their use may impair home compostability of the material.

Final coating preferably consists of tung oil in combination with pure pine turpentine oil (4:1). This coating is preferably applied at least twice from each side and left to dry completely in between each coat. This can be substituted or in addition to wax emulsion coating. Beeswax / Carnauba wax / rice bran wax with anionic polarity can be used for this process. Latex or biopolymers can be added to the coatings to enhance the abrasion resistance and moisture resistance.

Abaca fibre can be used instead of banana fibre. 0 to 100 % of banana fibre can be replaced by abaca fibre.

The banana fibre, the hemp fibre, the sisal fibre and bacterial cellulose can preferably come from 100% renewable sources where 50-60 wt. % of the material composition comes from agricultural waste streams - banana fibre and bacterial cellulose which is grown on agricultural waste from processing mature coconuts.

Any time before step I. banana fibre, the hemp fibre and sisal fibre can be mechanically or enzymatically extracted for the raw material (from stems in case of hemp and banana, from leaves in case of sisal)

Any time before step II. the bacterial cellulose Nata de Coco can be obtained by a fermentation process of *Acetobacter* strain of bacteria on water from mature coconut / secondary coconut milk media. Fermentation can be static or agitated, it takes 14-20 days; after the harvest bacterial cellulose is washed and sterilised. Preferably static fermentation is used due to the fact that it provides higher yield.

Before or after step I.a) the respective fibres can be soaked in water for 2 to 5 hours in order to shorten the delignification process following this step.

According to the preferred embodiment, the banana fibre, the hemp fibre and the sisal fibre in the step I.b) are cooked in the alkali solution for at least 1 hour, preferably 2 to 3 hours and then cooled to a room temperature. The alkali solution preferably has concentration 8 to 10 wt. %. Preferably the alkali agent is NaOH.

Alternatively, the fibres can be delignified in step I.b) enzymatically using a blend of known microbially produced enzymes.

The ratio of the banana pulp : hemp pulp : sisal pulp is preferably 50 : 35 : 15 to 60 : 35 : 5, most preferably 50 : 35 : 15 or 60 : 35 : 5.

According to the preferred embodiment, in step 3 or any time before step 4 the pulp mixture is dyed with natural plant-based dyes, such as extracts from indigo, marigold, brazilwood or madder. Preferred percentage of dye is 5-10 % of weight of fibre.

According to further preferred embodiment, the polysaccharide gum is, in the step IV., firstly dissolved in a water in a ratio 1:10 and stirred until the solution gels, subsequently the gel solution is added to the mixture.

According to further preferred embodiment, the pH mixture in the step VI. is adjusted within the range 5-8, higher the pH, darker the colour of the final product is achieved.

According to further preferred embodiment, the sheet obtained in the step VI. is left in the mould until the thickness of the sheet is 1 to 3 cm, preferably 1.5 to 2 cm, and in the step VII the sheet is subjected to pressure until the thickness of the sheet is 2 to 7 mm, preferably 4 mm.

According to further preferred embodiment, the sheet after step VIII. and before step IX. is treated with stearic acid in amounts of 2 to 15 % of the weight of the sheet. Preferably the process of esterification is used for crosslinking the stearic acid with cellulose. This step significantly improves the moisture barrier properties of the sheet which positively influences the wet abrasion and tensile strength properties and allows the material to lock the desired amount of moisture inside. The evaporation of moisture is prevented and desired softness retained.

According to further preferred embodiment, in step IX. The glycerol and IPA are applied on the sheets by spraying or blade coating or fogging.

According to further preferred embodiment, in step XI. The glucomannan solution is prepared by dispersing the powder of a starch extract from konjac root in the 20 -25°C water and bringing it to 90°C, the solution is applied to the sheet surface from both sides by blade coating.

According to further preferred embodiment, in step XII. the material is softened and textured by passing the sheet through a series of rollers with dull blades in between the rollers from at least 4 directions, 50 to 90 times from each direction, or until the desired level of flexibility and texture is achieved.

According to further preferred embodiment, in step XIV. the hydrophobic agent is selected from the group comprising Beeswax, Carnauba wax, rice bran wax, candelilla wax, soy wax and saturated fatty acid-based oil preferably the mixture of tung oil and pine turpentine oil at a ratio 4:1. Alternatively, coating based on PLA or other bio polymer can be applied. Coatings based on natural Latex can be applied.

One of the advantages of this method/biocomposite is that no ingredients of petroleum oil-based origin are used in the composition or coatings of the material, which gives the material the advantage of full home compostability. The material is also rendered vegan since no ingredients of animal origin were used in the formulation.

Material is suitable for products with a life span of 4 - 10 years, preferably with lower load applications such as handbags, backpacks, accessories, watch straps, light footwear, interior objects, stationary or pet leashes.

Mechanical properties like thickness and density of the material are tuneable (with varying amounts of plant fibre content and level of compression applied) which influences the tear and tensile strength.

**Table 1**

| Table 1: Shows how the change in composition affects mechanical properties of the sheet. | | | |
|---|---|---|---|
| **Ingredient** | **Quantity [% wt.]** | **Ultimate Tensile Strength (UTS) [MPa]** | **Notes** |
| Bacterial cellulose | 1 | 7-8 | |
| Bacterial cellulose | 15 | 10-14 | |

| **Ingredient** | **Content [% wt. ]** | **Moisture uptake after full immersion in water for 5 min. [%]** | |
|---|---|---|---|
| Stearic acid | 0 | 67 | |
| Stearic acid | 10 | 30 | |

| **Material name** | **Weight [g/m²]** | **Thickness [mm]** | |
|---|---|---|---|
| Biocomposite 1 | 500 | 0.5 -0.8 | |
| Biocomposite 2 | 800 | 1.5-1.8 | |

| **Material name** | **Compression [MPa]** | **Thickness [mm]** | |
|---|---|---|---|
| Biocomposite 1 | 0 | 0.95 | |
| Biocomposite 1 | 1 | 0.7 | |

Tensile strength of the material also depends on the length of the plant fibres are cut into as well as on the amount of polysaccharide gum used in the composite. For applications like footwear and upholstery (with higher load bearing requirement) higher values of tensile strength are desirable.

The temper and softness of the material is directly influenced by the amount of glycerol used in the material (a humectant) which should however not be higher than 30% of the weight of the sheet. For stability of these properties across environments with varying relative humidity % it is essential to apply effective moisture barriers such as hereby suggested esterification by stearic acid.

Softness is also influenced by the graining process; however, the effect of this without use of humectant and esterification with stearic acid would be reversible in unstable RH.

The bacterial cellulose bio-composite material according to the present invention is suitable for the various applications, among others also as:
- Watch strap:
   Materials for this application may vary according to the aesthetic preferences one chooses for the finished strap. However due to the frequent contact with human skin it is required that it has good resistance to wet and dry abrasion, stability in contact with human sweat and good degree of flexing endurance.
   For all the above-mentioned properties enhancement it is necessary to complete treatment by 8-10% stearic acid. Ideally 500-650 g/m² weight materials are recommended. 0.5-1% of natural latex content is also recommended in order to improve resistance to water uptake.
- Wallet:
   Materials with good abrasion resistance, flexing endurance and thickness of 0.3-1 mm are most suitable for this application. Other mechanical properties such as elastic elongation and tensile strength are of less significance for wallet making. 350-500 g/m² weights are selected to achieve the desired thickness of the material. The % of polysaccharide gum is maintained at 5-8%. Treatment of semi-finished material with 8-12% stearic acid is an optional step as this positively influences both material wet and dry abrasion resistance as well as Tensile strength (material is often split or skived at the edges). 350-400 g/m² materials are necessary to be backed up using gauze fabric in order to improve tear strength.
- Handbag:
   Handbags and materials used for making them is a very broad category that mainly depends on the selected design and method of production of the chosen design.
   Material weights from 500 to 800 can be used where 500 g/m² results in thinner, more pliable material, increasing g/m² leads to thicker material with higher TS and more structural character.
   Content % of glycerol can be adjusted to achieve material of desired softness from 10-15% in case of semi stiff - stiff temper to 25-30% to achieve semi soft to soft temper.
- Footwear upper:
   Footwear is an application where higher values of elastic elongation and TS are required. In order to increase elastic elongation % it is recommended to add 0.5-10% of natural latex in the biocomposite slurry prior to the sheet forming stage. Glycerol content shall be adjusted to 20-25% of the weight of the sheet and stearic acid treatment shall be completed at 8-10%. Material weights ranging from 500 to 650 g/m² are recommended. 0.5-5% of chitosan can be added instead of natural rubber latex to improve the moisture uptake and wet strength.

The effect of Glycerol on the moisture content in the material and also on the binding potential of fibres to bacterial cellulose:
Weight increase after glycerol application (in %): 15-30
Moisture content increase after glycerol application (in %): 5-15
Moisture content range with 5% glycerol: 5-10%
Moisture content range with 25-30% glycerol application: 15-25%

**Table 2**

| Table 2 shows the effect of the guar gum content on the properties of the biocomposite material according to the invention. | | | |
|---|---|---|---|
| **Guar gum content %** | **Load (N)** | **UTS (MPa)** | **Elastic elongation (%)** |
| 2.6 | 113.9 | 7.19 | 22.4 |
| 5 | 176.7 | 10.5 | 21 |
| 8 | 147.9 | 17.3 | 19.6 |
| 28 | 285.6 | 15.3 | 14.7 |

**Table 3**

| The table 3 shows how the treatment with stearic acid improve tensile strength of the biocomposite material. | | |
|---|---|---|
| **Sample** | **Tensile strength (UTS) after drying in oven at 80°C for 2 hours** | **Tensile strength (UTS) after 3 hours in Stability chamber at 30°C (MPa) & 70% RH** |
| | **(MPa)** | |
| semi-finished biocomposite sample | 11 | 17 |
| finished sample treated with stearic acid (10%) | 18 | 21 |

**Table 4**

| The table 4 shows the water-resistant properties of the glucomannan coating. | |
|---|---|
| **Stage of material processing** | **water droplet contact angle degree** |
| no coatings (post folding) | 0 |
| 1x glucomannan coating | 100 |
| all coatings (glucomannan, tung oil) | 95 |

### DETAILED DESCRIPTION

### Example 1:

### Method of preparation of a biocomposite sheet of 500 g/m² in dark indigo colour with enhanced tensile strength

### Fibre preparation:

Banana, hemp and sisal scutched fibres in their dried extracted form are cut to length of approximately 7 cm. Fibres are soaked (for min. 4 hours), cooked separately in 8% alkali solution for 1.5 hours and left to cool to a room temperature. Fibres are drained and washed until water runs clear.

Fibres are then pulped for 45 minutes (banana and sisal), hemp fibres require pulping for min. 2 hours using Hollander Beater separately for each fibre type until the degree of fibrillation is less than in case of kraft paper or paper. It is important to maintain long fibre length during the pulping process (15-20mm).

Degree of fibrillation is checked using the Schopper Riegler tester.

bacterial cellulose is pulped for 5 min a cycle using high speed mixers and/or ultrasonic homogenizers into a very fine pulp. The pH is checked and adjusted to 5.5.

### Biocomposite preparation:

The respective components are mixed at the following ratio: 42 % wt. of banana fibre, 29 % wt. of hemp fibre, 13 % wt. of sisal fibre and 16 % wt. of bacterial cellulose.

Further the mixture can be dipped into 5% wt. of indigo dye solution for 3-5 min depending on the desired intensity of the colour. The pH of the solution is 8-10.

25g (8%) of guar gum is dissolved in 1.5 l of water and poured into the mixture that is being continuously agitated.

The mixture is agitated for 20-30 min to ensure homogenous dispersion of the fibres in the matrix.

The mixture is diluted to 0.3% wt. with water. The pH is adjusted to 8-10.

### Sheet forming process:

The diluted solution of fibres and matrix is poured into a sheet forming mould. The water is filtered out through the bottom mesh using a mild vacuum and water recycled in the next sheet making process. After 25-35 min, when the water is removed to approximately 1.5% content of the initial volume, a sheet is removed from the mould. The weight of the sheet at that point is around 2-3 kg and its thickness is 1.5 to 2 cm. Sheet is further dewatered in hydraulic press under 1 MPa pressure to thickness of approximately 5 mm. The moisture content in the pressed sheet is 60-80%. It is placed on a mesh frame and transported to a drying facility.

### Sheet drying:

The sheet is dried at the temperature of approximately 35°C for 4-8 hours to achieve high tensile and tear strength of the material.

Material is then esterified in the solution of 10 % stearic acid in ethyl alcohol at the temperature of 70°C for 3 hours, to improve the water resistance of the material. Material is then washed with distilled water or alcohol and dried. It has been reported that materials with higher % of moisture locked in can display higher values of tensile strength. A sample untreated with stearic acid had tensile strength values 15-25% lower than the one with stearic acid treatment after full processing.

### Sheet stretching, softening and texturizing:

The sheet is left to dry and then treated with 20 % wt. solution of glycerol for both sides. The Sheet is left to dry.

A specially developed folding machine is employed to stretch the surface of the sheet and remove the warps. Folds have spacing of 2 cm in between each other. The sheet is folded in 4 directions - horizontally, vertically, and diagonally (2x).

Sheet is then pressed under pressure of 1-5 MPa at temperature 80 °C in a horizontal press.

A coating is prepared using glucomannan (99% purity starch extract from konjac root) powder and water (12 g per litre) by dispersing the powder in cold water and slowly bringing it to boil. The coating is applied to the surface of the sheet using a blade coating method from both sides.

Once dry the material is softened and texturized using a graining type of machine consisting of a series of rollers and a dull blade in between, the sheet is passed through the machine from 4 directions, 80-90 times in each direction.

### Sheet coating:

The sheet is coated with the glucomannan coating 2x from each side and let dry.

Final coating consists of tung oil in combination with pure pine turpentine oil (4:1).

This coating is applied twice from each side and left to dry completely in between each coat.

Mechanical properties of the final product:

| No. | mechanical property | value range |
|---|---|---|
| 1 | UTS | (load 159-250 N) 17-24 MPa |
| 2 | Elastic elongation (strain) | 13-18% |

### Example 2:

### Method of preparation of a biocomposite sheet of 650 g/m² in Cumin Yellow colour with enhanced elastic elongation

### Fibre preparation:

Fibre preparation is the same as in Example 1. Preferably enzymatically treated (delignified) banana fibres or freshly extracted banana fibres are used for the preparation as they display higher elastic elongation degree than alkali treated ones or once dried ones.

### Biocomposite preparation:

The respective components are mixed at the following ratio: 46 % wt. of banana fibre (preferably enzymatically treated as it yields fibres that have higher elastic elongation values), 27 % wt. of hemp fibre, 11 % wt. of sisal fibre and 15 % wt. of bacterial cellulose.

Further 5% wt. of pomegranate peel extract dye solution is added to the mixture and the mixture is agitated for 45 min.

20g (5%) of guar gum is dissolved in 1.5 l of water and poured into the mixture that is being continuously agitated.

The pH of the mixture is adjusted to 11 and 0.5% wt. a natural rubber latex solution with 0.03% ammonia is added and agitated. It has been found that low concentrations of Latex dispersed in the slurry positively influences the elastic elongation value of the biocomposite (by 5-10% increase in elastic elongation).

The mixture is agitated for 20-30 min to ensure homogenous dispersion of the fibres in the matrix.

The mixture is diluted to 0.3% wt. with water. The pH is maintained at 11.

### Sheet forming process:

### Same as in Example 1

### Sheet drying:

The sheet is dried at the temperature of approximately 35°C for 8 hours to achieve high tensile and tear strength of the material. Esterification process is optional at this stage but not preferred as it decreases elastic elongation value of the material (by 5-8%).

### Sheet stretching, softening and texturizing:

### Same as in Example 1.

### Sheet coating:

The sheet is coated with the glucomannan coating 2x from each side and let dry.

Final coating consists of tung oil in combination with pure pine turpentine oil (4:1).

This coating is applied twice from each side and left to dry completely in between each coat.

Mechanical properties of the final product:

| No. | mechanical property | value range |
|---|---|---|
| 1 | UTS | 14-17 MPa |
| 2 | Elastic elongation (strain) | 25-39 % |

### Example 3:

### Method of preparation of a biocomposite sheet of 800 g/m² in natural undyed colour with texture

### Fibre preparation:

Banana, hemp and sisal scutched fibres in their dried extracted form are cut to length of approximately 7 cm. Fibres are cooked separately in 9 % alkali solution for 1.5 hours and left to cool to a room temperature. Fibres are drained and washed until water runs clear.

Fibres are then pulped for 45 minutes (banana and sisal), hemp fibres require pulping for min. 2 hours using Hollander Beater separately for each fibre type until the degree of fibrillation is less than in case of kraft paper or paper. It is important to maintain long fibre length during the pulping process (15-20mm).

Degree of fibrillation is checked using the Schopper Riegler tester.

2.5% of H2O2 or 0.2% laccase enzyme can be added to the beater while beating the process to bleach the fibres and prepare a sheet of more uniform colour. When fibres have uniform colour over the surface it is beneficial for the aesthetic properties and visibility of the texture that is less visible and prominent on materials with surface colour that is not uniform throughout the area.

Bacterial cellulose is pulped for 5 min a cycle using high speed mixers and/or ultrasonic homogenizers into a very fine pulp. The pH is checked and adjusted to 5.5.

### Biocomposite preparation:

The respective components are mixed at the following ratio: 44 % wt. of banana fibre, 29 % wt. of hemp fibre, 12 % wt. of sisal fibre and 12 % wt. of bacterial cellulose.

24g (3%) of guar gum is dissolved in 1.5 l of water and poured into the mixture that is being continuously agitated.

The mixture is agitated for 20-30 min to ensure homogenous dispersion of the fibres in the matrix.

The mixture is diluted to 0.3% wt. with water. The pH is adjusted to 10.

### Sheet forming process:

### Same as in example 1.

### Sheet drying:

### Same as in example 1.

### Sheet stretching, softening and texturizing

The sheet is left to dry and then treated with 20 % wt. solution of glycerol for both sides. The Sheet is left to dry.

The surface of the sheet is stretched by a folding machine to remove the warps. Folds have spacing of 2 cm in between each other. The sheet is folded in 4 directions - horizontally, vertically, and diagonally (twice in each direction).

Sheet is then pressed under pressure of 1-5 MPa at temperature 80°C in a horizontal press.

A coating is prepared using glucomannan powder and water (12g per liter) by dispersing the powder in cold water and slowly bringing it to boil. The coating is applied to the surface of the sheet using a blade coating method from both sides.

Once dry the material is softened and texturised using a graining type of machine consisting of a series of rollers and a dull blade in between, the sheet is passed through the machine from 4 directions, 50-60 times in each direction.

A sheet is passed through a calendering machine bearing texturising pattern applying pressure of 1-5 MPa to achieve distinctive pattern.

### Sheet coating:

Material is coated with 1x glucomannan coating and let dry. Subsequently it is treated with anionic carnauba wax emulsion

Mechanical properties of the final product:

| No. | mechanical property | value range |
|---|---|---|
| 1 | UTS | 13-17 MPa (130-180 N load) |
| 2 | Elastic elongation (strain) | 12-16% |

## Claims

1. A bacterial cellulose bio-composite obtained by the following manufacturing process:
I.
a) cutting the banana fibre, the hemp fibre and the sisal fibre, each separately, to a length of 2 to 10 cm, preferably to length of 6 to 8 cm;
b) delignification of the banana fibre, the hemp fibre and the sisal fibre, each separately, either enzymatically or in the alkaline solution;
c) removing impurities from the delignified banana fibre, the hemp fibre and the sisal fibre, preferably washed with water;
d) pulping the banana fibre, the hemp fibre and the sisal fibre, each separately, mechanically or enzymatically to a length 5 - 50 mm;
II. pulping a bacterial cellulose to a length of 10-100 µm; and if necessary, adjusting pH of the pulp to 5 to 7, preferably to 5.5;
III. mixing the components obtained in the steps I. and II. in amounts:
- 35 to 60 wt. % of banana pulp,
- 20 to 45 wt.% of hemp pulp,
- 5 to 20 wt. % of sisal pulp, and
- 10 to 20 wt. % of bacterial cellulose pulp;
IV. adding 0,2 to 20 wt. % of polysaccharide cationic gum into the mixture, preferably 3 to 10 wt. %, the most preferably 5 wt. %;
V. stirring the mixture until homogenous dispersion of the fibres in the matrix (matrix being the binding part of the composite) is achieved;
VI. diluting the mixture with water to achieve 0.3 wt. % to 0.03 % of composite solution and pouring the solution of fibres and matrix in a sheet forming mould having a water-permeable bottom, and left there until the water is filtered out through the bottom until 25 to 15 % of the initial volume of the composite solution is achieved, preferably 15 %;
VII. further dewatering the sheet until the content of water is less than 80% of the sheet weight,
VIII. drying the sheet at the temperature up to 60 °C, preferably 20 to 50 °C until the overall moisture content in the sheet is lower than 10 wt.%, preferably 8-9 wt.%;
IX. treating the sheet with 5 to 30 wt. % humectant with respect to dry weight of sheet from both sides and letting the sheet absorb this solution, until the moisture content of the sheet increases to 20-30 wt %;
X. mechanical manipulation - multiple stretching and folding of the sheet in order for material to be pliable and without warps;
XI. treating both sides of the sheet with 10 - 30 wt.%, preferably 12 wt.%, water solution of sizing to improve tactile properties, preferably the tactile sizing is glucomannan, and drying the sheet;
XII. sheet is further mechanically softened and texturized, preferably using a graining machine,
XIII. performing step XI, at least once;
XIV. the dry sheet is optionally treated with hydrophobic agent.

2. The bacterial cellulose bio-composite according to claim 1, **characterised in that**, any time before step II. the bacterial cellulose Nata de Coco is obtained by a fermentation process of *Acetobacter* strain of bacteria on water from mature coconut / secondary coconut milk media.

3. The bacterial cellulose bio-composite according to any of the preceding claims, **characterised in that**, the banana fibre, the hemp fibre and the sisal fibre in the step I.b) are cooked in the alkali solution for at least 1 hour, preferably 2 to 3 hours and then cooled to a room temperature.

4. The bacterial cellulose bio-composite according to claims 1 or 3, **characterised in that**, the alkali solution in step I.b) has a concentration of 8 to 10 wt. %.

5. The bacterial cellulose bio-composite according to any of the preceding claims, **characterised in that**, the ratio of the banana pulp : hemp pulp : sisal pulp is 50 : 35 : 15 to 60 : 35 : 5.

6. The bacterial cellulose bio-composite according to any of the preceding claims, **characterised in that**, in the step III. the pulp mixture is dyed with natural plant-based dyes, such as extracts from indigo, marigold, brazilwood or madder, preferably in percentage 5-10% of the weight of the fibre.

7. The bacterial cellulose bio-composite according to any of the preceding claims, **characterised in that**, in the step IV. the polysaccharide gum is firstly dissolved in water in a ratio 1:10 and stirred until the solution gels, subsequently the gel is added to the mixture.

8. The bacterial cellulose bio-composite according to any of the preceding claims, **characterised in that**, in the step VI. the sheet is left in the mould until the thickness of the sheet is 1 to 3 cm, preferably 1.5 to 2 cm, and in step VII the sheet is subjected to pressure until the thickness of the sheet is 2 to 7 mm, preferably 4 mm.

9. The bacterial cellulose bio-composite according to any of the preceding claims, **characterised in that**, after step VIII and before step IX., the sheet is treated with stearic acid in amount of 2 to 15 % of the weight of the sheet, preferably the process of esterification is used for crosslinking the stearic acid with cellulose.

10. The bacterial cellulose bio-composite according to any of the preceding claims, **characterised in that**, in step IX. the humectant is applied on the sheets by spraying or blade coating or fogging, wherein the humectant is preferably glycerol or isopropyl alcohol or combination thereof.

11. The bacterial cellulose bio-composite according to any of the preceding claims, **characterised in that**, in the step XI. the glucomannan solution is prepared by dispersing the powder of a starch extract from konjac root in the water of temperature of 20 to 25 °C and bringing it to the temperature of 90 °C, the solution is applied to the sheet from both sides.

12. The bacterial cellulose bio-composite according to any of the preceding claims, **characterised in that**, in the step XII. the material is softened and texturized by passing the sheet through a series of rollers with dull blades in between the rollers from at least 4 directions, 50 to 90 times from each direction, or until the desired level of flexibility and texture is achieved.

13. The bacterial cellulose bio-composite according to any of the preceding claims, **characterised in that**, in the step XIV., the hydrophobic agent is selected from the group comprising beeswax, carnauba wax, rice bran wax, candelilla wax, soy wax and saturated fatty acid-based oil preferably the mixture of tung oil and pine turpentine oil at a ratio 4:1, alternatively, coating based on polylactic acid polymer or natural latex can be applied.

14. An article made of or comprising the bacterial cellulose bio-composite according to the claims 1 to 13.

## Patentansprüche

1. Bakterienzellulose-Biokomposit, erhalten durch folgendes Herstellungsverfahren:
I.
a) Schneiden der Bananenfaser, der Hanffaser und der Sisalfaser, jeweils separat, auf eine Länge von 2 bis 10 cm, vorzugsweise auf eine Länge von 6 bis 8 cm;
b) Delignifizierung der Bananenfaser, der Hanffaser und der Sisalfaser, jeweils separat, entweder enzymatisch oder in alkalischer Lösung;
c) Entfernen von Verunreinigungen aus der delignifizierten Bananenfaser, Hanffaser und Sisalfaser, vorzugsweise durch Waschen mit Wasser;
d) Aufschluss der Bananenfaser, der Hanffaser und der Sisalfaser, jeweils separat, mechanisch oder enzymatisch auf eine Länge von 5-50 mm;
II. Aufschluss einer Bakterienzellulose auf eine Länge von 10-100 µm und gegebenenfalls Einstellung des pH-Wertes des Zellstoffs auf 5 bis 7, vorzugsweise 5,5;
III. Mischen der in den Schritten I. und II. erhaltenen Komponenten in folgenden Mengen:
• 35 bis 60 Gew.-% Bananenzellstoff,
• 20 bis 45 Gew.-% Hanfzellstoff,
• 5 bis 20 Gew.-% Sisalzellstoff, und
• 10 bis 20 Gew.-% Bakterienzellulosezellstoff;
IV. Zugabe von 0,2 bis 20 Gew.-% eines kationischen Polysaccharid-Gummis in die Mischung, vorzugsweise 3 bis 10 Gew.-%, am meisten bevorzugt 5 Gew.-%;
V. Rühren der Mischung, bis eine homogene Dispersion der Fasern in der Matrix (wobei die Matrix den bindenden Teil des Komposits bildet) erreicht ist;
VI. Verdünnen der Mischung mit Wasser, bis eine Konzentration von 0,3 bis 0,03 Gew.-% der Kompositlösung erreicht ist, und Eingießen der Lösung aus Fasern und Matrix in eine Bogengießform mit wasserdurchlässigem Boden und Belassen derselben, bis das Wasser durch den Boden abgefiltert ist, bis 25 bis 15 % des ursprünglichen Volumens der Kompositlösung erreicht sind, vorzugsweise 15 %;
VII. weiteres Entwässern des Bogens, bis der Wassergehalt weniger als 80 % des Bogen-gewichts beträgt;
VIII. Trocknen des Bogens bei Temperaturen bis 60 °C, vorzugsweise 20-50 °C, bis der Gesamtfeuchtigkeitsgehalt im Bogen unter 10 Gew.-% liegt, vorzugsweise 8-9 Gew.-%;
IX. Behandeln des Bogens mit 5 bis 30 Gew.-% Feuchthaltemittel, bezogen auf das Trockengewicht des Bogens, von beiden Seiten und Zulassen, dass der Bogen diese Lösung absorbiert, bis der Feuchtigkeitsgehalt des Bogens 20-30 Gew.-% beträgt;
X. Mechanische Bearbeitung - mehrfaches Strecken und Falten des Bogens, damit das Material biegsam ist und keine Verwerfungen aufweist;
XI. Behandeln beider Seiten des Bogens mit 10-30 Gew.-%, vorzugsweise 12 Gew.-%, einer wässrigen Lösung eines Leimungsmittels zur Verbesserung der haptischen Eigenschaften, vorzugsweise handelt es sich um Glucomannan, und Trocknen des Bogens;
XII. Weiteres mechanisches Erweichen und Texturieren des Bogens, vorzugsweise unter Verwendung einer Narbungsmaschine;
XIII. Erneute Durchführung von Schritt XI mindestens einmal;
XIV. Optionales Behandeln des trockenen Bogens mit einem hydrophoben Mittel.

2. Bakterienzellulose-Biokomposit nach Anspruch 1, **dadurch gekennzeichnet, dass** die Bakterienzellulose (Nata de Coco) jederzeit vor Schritt II durch einen Fermentationsprozess von Acetobacter-Bakterienstämmen auf Wasser aus reifen Kokosnüssen oder sekundärer Kokosmilch gewonnen wird.

3. Bakterienzellulose-Biokomposit nach einem der vorhergehenden Ansprüche, **dadurch gekennzeichnet, dass** die Bananen-, Hanf- und Sisalfasern in Schritt I.b) mindestens 1 Stunde, vorzugsweise 2 bis 3 Stunden, in alkalischer Lösung gekocht und anschließend auf Raumtemperatur abgekühlt werden.

4. Bakterienzellulose-Biokomposit nach Anspruch 1 oder 3, **dadurch gekennzeichnet, dass** die alkalische Lösung in Schritt I.b) eine Konzentration von 8 bis 10 Gew.-% aufweist.

5. Bakterienzellulose-Biokomposit nach einem der vorhergehenden Ansprüche, **dadurch gekennzeichnet, dass** das Verhältnis von Bananenzellstoff : Hanfzellstoff : Sisalzellstoff 50 : 35 : 15 bis 60 : 35 : 5 beträgt.

6. Bakterienzellulose-Biokomposit nach einem der vorhergehenden Ansprüche, **dadurch gekennzeichnet, dass** in Schritt III der Zellstoff mit natürlichen pflanzlichen Farbstoffen wie Extrakten aus Indigo, Ringelblume, Brasilholz oder Krapp gefärbt wird, vorzugsweise in einem Anteil von 5-10 % des Fasergewichts.

7. Bakterienzellulose-Biokomposit nach einem der vorhergehenden Ansprüche, **dadurch gekennzeichnet, dass** in Schritt IV das Polysaccharidgummi zunächst in Wasser im Verhältnis 1:10 gelöst und gerührt wird, bis eine Gelierung eintritt, und dass anschließend die Gellösung der Mischung zugesetzt wird.

8. Bakterienzellulose-Biokomposit nach einem der vorhergehenden Ansprüche, **dadurch gekennzeichnet, dass** in Schritt VI der Bogen in der Form belassen wird, bis die Dicke 1-3 cm, vorzugsweise 1,5-2 cm, beträgt, und dass in Schritt VII der Bogen einem Druck ausgesetzt wird, bis die Dicke 2-7 mm, vorzugsweise 4 mm, beträgt.

9. Bakterienzellulose-Biokomposit nach einem der vorhergehenden Ansprüche, **dadurch gekennzeichnet, dass** der Bogen nach Schritt VIII und vor Schritt IX mit Stearinsäure in einer Menge von 2-15 % des Bogen-gewichts behandelt wird, wobei vorzugsweise eine Veresterung zur Vernetzung der Stearinsäure mit der Zellulose durchgeführt wird.

10. Bakterienzellulose-Biokomposit nach einem der vorhergehenden Ansprüche, **dadurch gekennzeichnet, dass** in Schritt IX das Feuchthaltemittel durch Sprühen, Klingenbeschichtung oder Vernebelung aufgetragen wird, wobei das Feuchthaltemittel vorzugsweise Glycerin oder Isopropylalkohol oder eine Kombination davon ist.

11. Bakterienzellulose-Biokomposit nach einem der vorhergehenden Ansprüche, **dadurch gekennzeichnet, dass** in Schritt XI die Glucomannan-Lösung hergestellt wird, indem das Pulver eines Stärkextrakts aus der Konjakwurzel in Wasser bei 20-25 °C dispergiert und auf 90 °C erhitzt wird, und dass die Lösung auf beide Seiten des Bogens aufgetragen wird.

12. Bakterienzellulose-Biokomposit nach einem der vorhergehenden Ansprüche, **dadurch gekennzeichnet, dass** in Schritt XII das Material erweicht und texturiert wird, indem der Bogen durch eine Serie von Walzen mit stumpfen Klingen zwischen den Walzen in mindestens 4 Richtungen, 50-90 Mal pro Richtung, geführt wird oder bis das gewünschte Maß an Flexibilität und Textur erreicht ist.

13. Bakterienzellulose-Biokomposit nach einem der vorhergehenden Ansprüche, **dadurch gekennzeichnet, dass** in Schritt XIV das hydrophobe Mittel aus der Gruppe ausgewählt ist, die Bienenwachs, Carnaubawachs, Reiskleiewachs, Candelillawachs, Sojawachs und auf gesättigten Fettsäuren basierendes Öl umfasst, vorzugsweise eine Mischung aus Tungöl und Kiefernbalsamterpentinöl im Verhältnis 4:1, alternativ eine Beschichtung auf Basis von Polymilchsäurepolymer oder natürlichem Latex.

14. Artikel, hergestellt aus oder umfassend das Bakterienzellulose-Biokomposit nach den Ansprüchen 1 bis 13.

## Revendications

1. Biocomposite de cellulose bactérienne, obtenu par un procédé de fabrication comprenant:
I.
a) la coupe des fibres de bananier, de chanvre et de sisal, chacune séparément, à une longueur de 2 à 10 cm, de préférence 6 à 8 cm;
b) la délignification des fibres de bananier, de chanvre et de sisal, chacune séparément, soit par voie enzymatique, soit dans une solution alcaline;
c) l'élimination des impuretés desdites fibres délignifiées, de préférence par lavage à l'eau ;
d) le défibrage desdites fibres de bananier, de chanvre et de sisal, chacune séparément, par voie mécanique ou enzymatique, à une longueur de 5 à 50 mm;
II. le défibrage d'une cellulose bactérienne à une longueur de 10 à 100 µm et, le cas échéant, l'ajustement du pH de la suspension de cellulose à 5 à 7, de préférence 5,5;
III. le mélange des composants obtenus aux étapes I et II, dans les proportions suivantes:
• 35 à 60 % en poids de pâte de bananier,
• 20 à 45 % en poids de pâte de chanvre,
• 5 à 20 % en poids de pâte de sisal, et
• 10 à 20 % en poids de pâte de cellulose bactérienne;
IV. l'ajout de 0,2 à 20 % en poids d'une gomme polysaccharidique cationique au mélange, de préférence 3 à 10 %, et plus préférablement 5 %;
V. l'agitation du mélange jusqu'à obtention d'une dispersion homogène des fibres dans la matrice, ladite matrice constituant la partie liant du composite;
VI. la dilution du mélange avec de l'eau jusqu'à une concentration de 0,3 à 0,03 % en poids de solution composite, puis le coulage de la solution de fibres et de matrice dans un moule de formation de feuille à fond perméable à l'eau, et le maintien jusqu'à ce que l'eau soit filtrée à travers le fond, jusqu'à ce que 25 à 15 % du volume initial de la solution composite soit atteint, de préférence 15 %;
VII. le drainage complémentaire de la feuille jusqu'à ce que la teneur en eau soit inférieure à 80 % du poids total de la feuille;
VIII. le séchage de la feuille à une température allant jusqu'à 60 °C, de préférence 20 à 50 °C, jusqu'à ce que la teneur totale en humidité de la feuille soit inférieure à 10 % en poids, de préférence 8-9 %;
IX. le traitement de la feuille avec 5 à 30 % en poids d'agent humectant, basé sur le poids sec de la feuille, des deux côtés, et l'absorption de ladite solution par la feuille jusqu'à ce que la teneur en humidité atteigne 20-30 %;
X. une manipulation mécanique - pliage et étirement répétés de la feuille - de manière à obtenir un matériau souple et exempt de gauchissements;
XI. le traitement des deux faces de la feuille avec 10 à 30 %, de préférence 12 %, d'une solution aqueuse d'agent d'encollage, de préférence du glucomannane, puis le séchage de la feuille;
XII. l'assouplissement et la texturation ultérieurs de la feuille par voie mécanique, de préférence à l'aide d'une machine à grainage;
XIII. la répétition au moins une fois de l'étape XI;
XIV. le traitement optionnel de la feuille sèche avec un agent hydrophobe.

2. Biocomposite de cellulose bactérienne selon la revendication 1, **caractérisé en ce que** la cellulose bactérienne (Nata de Coco) est produite, avant l'étape II, par fermentation de souches d'Acetobacter sur de l'eau issue de noix de coco mûres ou du lait de coco secondaire.

3. Biocomposite de cellulose bactérienne selon l'une quelconque des revendications précédentes, **caractérisé en ce que** les fibres de bananier, de chanvre et de sisal à l'étape I.b) sont bouillies au moins 1 h, de préférence 2-3 h, dans une solution alcaline, puis refroidies à température ambiante.

4. Biocomposite de cellulose bactérienne selon la revendication 1 ou 3, **caractérisé en ce que** la solution alcaline à l'étape I.b) a une concentration de 8-10 % en poids.

5. Biocomposite de cellulose bactérienne selon l'une quelconque des revendications précédentes, **caractérisé en ce que** le rapport pâte de bananier : pâte de chanvre : pâte de sisal est de 50 : 35 : 15 à 60 : 35 : 5.

6. Biocomposite de cellulose bactérienne selon l'une quelconque des revendications précédentes, **caractérisé en ce que** la pâte obtenue à l'étape III est colorée avec des colorants végétaux tels que des extraits d'indigo, de calendula, de bois de Brésil ou de garance, dans une proportion de 5-10 % du poids des fibres.

7. Biocomposite de cellulose bactérienne selon l'une quelconque des revendications précédentes, **caractérisé en ce que** la gomme polysaccharidique est d'abord dissoute dans de l'eau dans un rapport 1:10 et agitée jusqu'à gélification, puis la solution gélifiée est ajoutée au mélange à l'étape IV.

8. Biocomposite de cellulose bactérienne selon l'une quelconque des revendications précédentes, **caractérisé en ce que** la feuille obtenue à l'étape VI est laissée dans le moule jusqu'à une épaisseur de 1-3 cm, de préférence 1,5-2 cm, et à l'étape VII pressée jusqu'à 2-7 mm, de préférence 4 mm.

9. Biocomposite de cellulose bactérienne selon l'une quelconque des revendications précédentes, **caractérisé en ce que** la feuille, après l'étape VIII et avant l'étape IX, est traitée avec 2-15 % en poids d'acide stéarique, de préférence par estérification.

10. Biocomposite de cellulose bactérienne selon l'une quelconque des revendications précédentes, **caractérisé en ce que** l'agent humectant de l'étape IX est appliqué par pulvérisation, couchage à la lame ou nébulisation, et comprend du glycérol, de l'isopropanol ou une combinaison de ceux-ci.

11. Biocomposite de cellulose bactérienne selon l'une quelconque des revendications précédentes, **caractérisé en ce que** la solution de glucomannane de l'étape XI est préparée en dispersant la poudre d'amidon de racine de konjac dans de l'eau à 20-25 °C, chauffée à 90 °C, et appliquée sur les deux faces de la feuille.

12. Biocomposite de cellulose bactérienne selon l'une quelconque des revendications précédentes, **caractérisé en ce que** la feuille est assouplie et texturée à l'étape XII en la faisant passer à travers une série de rouleaux avec lames émoussées, dans au moins 4 directions, 50-90 fois par direction.

13. Biocomposite de cellulose bactérienne selon l'une quelconque des revendications précédentes, **caractérisé en ce que** l'agent hydrophobe de l'étape XIV est choisi parmi la cire d'abeille, la cire de carnauba, la cire de son de riz, la cire de candelilla, la cire de soja et les huiles à base d'acides gras saturés, de préférence un mélange huile de tung/essence de térébenthine 4:1, ou encore du PLA, du latex naturel ou un biopolymère.

14. Article fabriqué à partir du biocomposite de cellulose bactérienne selon l'une quelconque des revendications 1 à 13.
